# EUROPÄISCHE PATENTSCHRIFT

(11) **EP 0 606 057 B1**
(45) Veröffentlichungstag und Bekanntmachung des Hinweises auf die Patenterteilung: **18.06.1997**
(21) Anmeldenummer: 94100014.3
(22) Anmeldetag: 03.01.1994
(51) Int. Cl.: C07C 69/618, C07C 67/347

(54) **Verfahren zur Herstellung von aromatischen Olefinen**
Process for the preparation of aromatic olefins
Procédé pour la préparation d'oléfines aromatiques

(30) Priorität: 07.01.1993 DE 4300194
(43) Veröffentlichungstag der Anmeldung: 13.07.1994
(73) Patentinhaber: HOECHST AKTIENGESELLSCHAFT, 65929 Frankfurt am Main (DE)
(72) Erfinder: Beller, Matthias, Dr., D-65527 Niedernhausen (DE); Fischer, Hartmut, D-65719 Hofheim (DE); Strutz, Heinz, Dr., D-61250 Usingen (DE)

(56) Entgegenhaltungen:
- EP-A- 0 508 264
- GB-A- 1 301 996
- BULLETIN OF THE CHEMICAL SOCIETY OF JAPAN, Band 52, Nr. 9, 1979 K. KIKUKAWA et al. "Reaction of Diazonium Salts with Transition Metals-II. Palladium catalyzed Arylation of Ethylene with Arenediazonium Salts" Seiten 2609-2610
- TETRAHEDRON, Band 37, 1981, Oxford-New York-Toronto-Paris -Sidney-Frankfurt. K.KIKUKAWA et al. "Reaction of Diazonium Salts with Transition Metalls -III. Palladium(o)-catalyzed Arylation of unsaturated compounds with Arenediazonium Salts" Seiten 31-36
- CHEMISTRY LETTERS, Nr. 1, 1977 K. KIKUKAWA et al. "Reaction of Diazonium Salts with Transition Metals. I. Arylation of Olefins with Arenediazonium Salts catalyzed by zero valent Palladium" Seiten 159-162

## Beschreibung

Die Erfindung betrifft ein verbessertes Verfahren zur Herstellung von aromatischen Olefinen durch katalysierte Olefinierung der entsprechenden Aryldiazoniumsalze in Gegenwart heterogener Palladiumkatalysatoren.

Die Herstellung aromatischer Olefine durch katalysierte Olefinierung von Aryldiazoniumsalzen in Gegenwart eines homogenen Palladiumkatalysators ist eine relativ neue Synthesemethode. Für eine technische Umsetzung sind die bisher in der Literatur beschriebenen Verfahren zur Herstellung aromatischer Olefine auf Basis von Diazoniumsalzen in Gegenwart eines Katalysators aufgrund des Fehlens einer effektiven Katalysatorwiedergewinnung jedoch ungeeignet.

Von K. Kikukawa et al. (Chem. Lett., 1977, 159; Bull. Chem. Soc., 1979, 52, 2609; Tetrahedron, 1981, 37, 31) wird die Vinylierung von Aryldiazoniumsalzen in Gegenwart von homogenen Palladium(0)komplexen und einer Base beschrieben. Die dort beschriebenen Reaktionen gelingen lediglich in guten Ausbeuten bei Verwendung von besonders teurem Bisbenzylidenpalladium(0) als Katalysator in Gegenwart überstöchiometrischer Mengen Base. Außerdem werden relativ große Mengen des Palladiumkomplexes eingesetzt (2 mol%, bezogen auf Aryldiazoniumsalz), der nach der Reaktion verworfen werden muß.

Gemäß W. Yong et al. (Synthesis, 1991, 967) gelingt die Umsetzung von Aryldiazoniumsalzen mit Camphen in Gegenwart von Palladiumacetat in Ethanol überwiegend nur in mäßigen Ausbeuten. Ungünstig für eine technische Umsetzung ist hierbei die Verwendung von Palladiumacetat als Katalysator, das nach der Reaktion aufwendig gereinigt werden muß, um wieder verwendet werden zu können. Die Reaktion ist bisher lediglich für Camphen beschrieben.

Nach J. H. Xu et al. (Youji Huaxue, 1987, 452; C.A., 1988, 109, 92408) kann man Aryldiazoniumsalze auch mit Lithiumtetrachloropalladat als Katalysator in Methanol mit Acrylsäure und Acrylsäureestern umsetzen. Auch bei diesem Verfahren ist eine Wiederverwendung des Katalysators technisch nur sehr aufwendig zu realisieren.

Die EP-A 0 508 264 betrifft ein Verfahren zur Herstellung von Arylolefinen durch Umsetzung von Aryldiazoniumsalzen mit Olefinen in Gegenwart eines Palladium-Katalysators. Die Verwendung von heterogenen PalladiumKatalysatoren wird zwar erwähnt (vergl. EP-A 0 508 264 A1 Seite 4, Zeilen 2 bis 3), sie wird allerdings experimentell lediglich in einem Fall, nämlich bei der Umsetzung von Anilin-2-sulfonsäure mit Ethylen zu Styrol-2-sulfonsäure (Beispiel 6), belegt. Wie ein Vergleich mit dem unter Einsatz von Pd(OAc)₂ durchgeführten Beispiel 4 zeigt, nimmt jedoch die Ausbeute bei Verwendung eines Palladium-Trägerkatalysators (10 % Pd auf Kohle) erheblich ab (Beispiel 4: 87 % Ausbeute; Beispiel 6: 74 % Ausbeute). In beiden Beispielen wird die Umsetzung unter Zusatz einer Base, die bezogen auf die Anilin-2-sulfonsäure im Überschuß eingesetzt wird, durchgeführt. Wie der experimentelle Befund beweist (siehe Vergleichsversuch im experimentellen Teil), läßt sich die in Beispielen der EP-A 0 508 264 praktizierte Arbeitsweise die Aryldiazoniumsalze in situ herzustellen und anschließend weiterzuverarbeiten, nicht generell auf eine Verwendung von Palladium enthaltenden Trägerkatalysatoren übertragen. Der Vergleichsversuch belegt, daß das gewünschte aromatische Olefin nicht einmal in kleinen Mengen gebildet wird.

Es bestand somit ein erhebliches Bedürfnis nach einem allgemeinen Verfahren zur katalytischen Olefinierung von Aryldiazoniumsalzen, das aromatische Olefine, insbesondere Zimtsäuren und deren Derivate sowie Styrole und Stilbene in möglichst hoher Ausbeute ohne störende Verunreinigungen und in einfacher Weise liefert und das zusätzlich eine einfache technische Umsetzung erlaubt. Insbesondere sollte eine Methode gefunden werden, bei welcher ein einfaches stabiles Katalysatorsystem verwendet werden kann, das technisch rückgewinnbar ist und dennoch eine ähnliche Aktivität wie herkömmliche homogene Palladiumkatalysatoren aufweist.

Es wurde nun überraschenderweise gefunden, daß man aromatische Olefine der allgemeinen Formel I 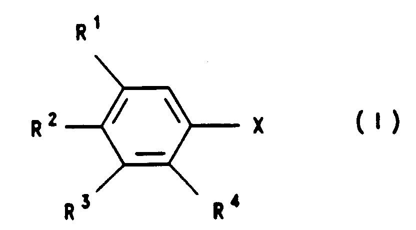 in welcher R¹, R², R³, unabhängig voneinander, Wasserstoff, Alkyl(C₁-C₈), Alkoxy (C₁-C₅), Phenyl, Fluor, Chlor, Brom, -OH, -NO₂, -CN, -CHO,
- CO(Alkyl)C₁-C₄),
-CO Phenyl, -COO Alkyl(C₁-C₄), -OCO Alkyl(C₁-C₄), -NHCO Alkyl(C₁-C₄), - CF₃, -NH₂, -NH Alkyl(C₁-C₄) oder -N(Alkyl(C₁-C₄))₂ und
R⁴ Wasserstoff, Alkyl(C₁-C₈), Phenyl, Fluor, Chlor, Brom, -OH,- NO₂, -CN, -CHO oder -OCO Alkyl(C₁-C₄) bedeuten, wobei die Alkyl- und die Alkoxygruppe geradkettig oder verzweigt sein können
X Alkenyl (C₂-C₁₂), Cycloalkenyl(C₄-C₈) oder die Gruppierung 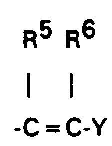 bedeutet, worin R⁵ und R⁶ unabhängig voneinander Wasserstoff oder Methyl, und Y -Phenyl, -CN, -COOH, -COO Alkyl(C₁-C₁₂), -COO Phenyl, - -CON(Alkyl(C₁-C₁₂))₂, -CONHAlkyl(C₁-C₁₂), 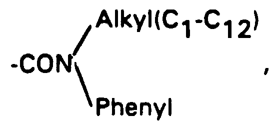 -CONH₂, -CON(Phenyl)₂, -COAlkyl(C₁-C₁₂), -CO Phenyl, -O Alkyl(C₁-C₁₂), -O Phenyl oder den Rest 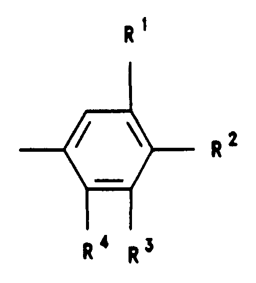 darstellen, worin R¹ - R⁴ die vorstehend genannten Bedeutungen haben, in vorteilhafter Weise herstellen kann, indem man ein Aryldiazoniumsalz der allgemeinen Formel (2) 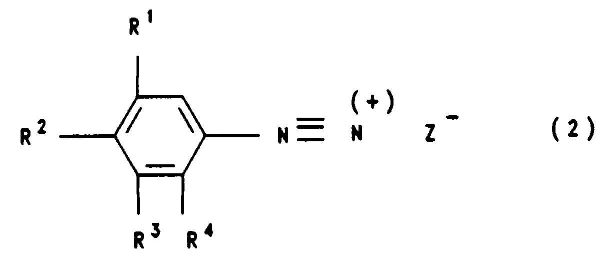 in welcher R¹, R², R³, R⁴ die vorstehend genannten Bedeutungen haben und Z das Äquivalent eines Anions einer organischen oder anorganischen Säure mit einem pKₐ-Wert kleiner als 7 darstellt, mit einem Olefin der allgemeinen Formel (3)

HX (3)

in welcher X die vorstehend genannte Bedeutung hat, in Gegenwart katalytischer Mengen eines heterogenen Palladiumkatalysators in einem organischen Lösungsmittel bei Temperaturen von -20 °C bis 150 °C, vorzugsweise 0 °C bis 100 °C, umsetzt.

Unter Aryldiazoniumsalzen werden Aryldiazoniumsalze als solche, insbesondere in reiner Form, beispielsweise in kristallinem Zustand, nicht jedoch in situ hergestellte Aryldiazoniumsalze verstanden.

Stellt X in der vorstehend genannten Formel (3) eine Alkenyl(C₃-C₁₂)- oder Cycloalkenyl(C₄-C₈)-gruppe dar, so kann es bei der Umsetzung zu einer Verschiebung der Doppelbindung kommen, wodurch in der Regel Isomerengemische entstehen.

Hinsichtlich der Bedeutung von R⁵ und R⁶ ist es vorteilhaft, wenn mindestens einer der Reste R⁵ und R⁶ ein Wasserstoffatom darstellt.

Die Substituenten R¹, R², R³ und R⁴ können beispielsweise eine Methyl-, Ethyl-, n-Propyl-, Isopropyl-, n-Butyl-, sek.-Butyl-, t-Butyl-, n-Pentyl-, 2-Pentyl-, n-Hexyl-, n-Heptyl-, n-Octyl- oder n-Decylgruppe, ferner eine Methoxy-, Ethoxy-, n-Propoxy-, Isopropoxy, n-Butoxy-, n-Pentyloxy-, Hexyloxy-, Octyloxy-, Decyloxy- oder Dodecyloxygruppe, ferner eine Carbonsäuremethyl-, -ethyl-, n-propyl-, isopropyl-, n-butyl- oder -sek. butylgruppe, außerdem eine Acetamid-, Propionamid-, Butyramid- oder Valeramidgruppe, ferner eine N,N-Dimethylamino-, N,N-Diethylamino-, N,N-Di-n-propylamino-, N,N-Di-n-butylamino-, N-Methyl-N-ethylamino- und N-Methyl-N-n-propylaminogruppe, schliesslich eine Acetoxy-, Propionyloxy- oder Butyryloxygruppe darstellen.

Die in den Gruppen von Y enthaltenen Alkylgruppen sind vorzugsweise Methyl-, Ethyl-, n-Butyl- oder 2-Ethyl-hexyl-gruppen.

Die Alkylgruppen für R¹ - R³ sind vorzugsweise geradkettig und enthalten vorzugsweise 1 - 4, besonders bevorzugt 1 - 2 Kohlenstoffatome. Die bevorzugten Alkoxygruppen für R¹ - R³ sind die Methoxy- und Ethoxygruppe.

Als Verbindungen der genannten Formel (2) verwendet man bevorzugt solche, bei denen R⁴ Wasserstoff, und R¹ bis R³ unabhängig voneinander Wasserstoff, Phenyl, Alkyl(C₁-C₃), insbesondere Methyl, Methoxy, Ethoxy, Acetoxy, -NO₂, -CN, -CHO, -Cl, -NHCOAlkyl(C₁-C₂), -COOAlkyl(C₁-C₂), -COAlkyl(C₁-C₂), -CO-Phenyl oder -N(Alkyl(C₁-C₂))₂ bedeuten.

Besonders bevorzugt verwendet man Verbindungen der Formel (2), worin R¹, R³ und R⁴ Wasserstoff oder Methyl, und R² Wasserstoff, Methyl, Phenyl, Methoxy, Ethoxy, Acetoxy, -CN, -NO₂, -COOAlkyl(C₁-C₂), -CHO, -Cl oder -COCH₃ darstellen.

Als Verbindungen der genannten allgemeinen Formel (3) verwendet man vorzugsweise solche, bei denen X Alkenyl (C₂-C₈), Cyclopentenyl oder Cyclohexenyl bedeutet oder die Gruppierung 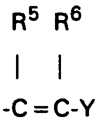 darstellt, in welcher einer der Substituenten R⁵, R⁶ ein Wasserstoffatom und der andere eine Methylgruppe darstellt, oder beide je ein Wasserstoffatom bedeuten, und Y Phenyl, -CN, -CON(Phenyl)₂, -COOH, -CONH₂, -CON(Alkyl)(C₁-C₁₂))₂, 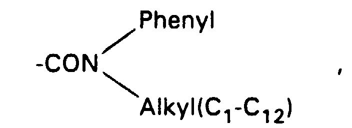 -COO(Alkyl)(C₁-C₁₂)
insbesondere aber CO₂-Methyl, CO₂-Ethyl, CO₂-2-Ethylhexyl bedeutet.

Besonders bevorzugt verwendet man als Verbindungen der genannten Formel (3) Styrol, Ethylen, Propylen, Acrylsäuremethyl-, -ethyl-, -butyl- oder -2-ethylhexylester.

Am bevorzugsten ist die Herstellung von 4-Methoxy, 4-Acetyl-, 4-Formyl-, 4-Nitro- und 4-Cyanozimtsäure-butylester oder -2-ethylhexylester durch Umsetzung von 4-Methoxy-, 4-Acetyl-, 4-Formyl-, 4-Nitro- oder 4-Cyanobenzoldiazoniumsalz mit dem enstprechenden Acrylsäureester, sowie die Herstellung von 4-Cyanostilben oder 4,4'-Dicyanostilben durch Umsetzung von 4-Cyanobenzoldiazoniumsalz mit Styrol bzw. Ethylen.

Der Palladiumkatalysator wird zweckmäßigerweise auf einem Trägermaterial, wie beispielsweise Aktivkohle, Calciumcarbonat, Bariumsulfat, Bimsstein, Tonerde, Kieselgur, Kieselgel und/oder Aluminiumoxid angewendet. Bevorzugt wird Palladium auf Aktivkohle oder Aluminiumoxid als Trägermaterial zur Anwendung gebracht.

Es ist zweckmäßig, einen Palladium-Trägerkatalysator anzuwenden, der 1 bis 20 Gew.-%, vorzugsweise 2 bis 10 Gew.-% Palladium, bezogen auf das Trägermaterial, enthält.

Hinsichtlich des Mengenverhältnisses Palladium zu Aryldiazoniumsalz ist es zweckmäßig, 0,001 bis 20 mol%, vorzugsweise 0,1 bis 1 mol% Palladium, bezogen auf das Aryldiazoniumsalz, anzuwenden.

Was das Mengenverhältnis der Reaktanten anbelangt, so ist es zweckmäßig, 1 mol Aryldiazoniumsalz der allgemeinen Formel (2) mit 0,2 bis 10 mol, vorzugsweise 0,4 bis 5 mol, besonders bevorzugt etwa 0,6 bis etwa 4 mol Olefin der allgemeinen Formel (3) zur Umsetzung zu bringen.

An in Frage kommenden Säureresten Z seien beispielsweise BF₄⁻, HSO₄⁻, PF₆⁻, SO₄²⁻, Cl⁻, CH₃COO⁻, Aryl-SO₃-, wobei der Arylrest substituiert sein kann, beispielsweise Naphthalinsulfonat oder Phenylsulfonat genannt.

Was die technische Verwendbarkeit der verfahrensgemäss erhältlichen Verbindungen anbelangt, so finden Stilbene als optische Aufheller, und Zimtsäureester beispielsweise als UV-Absorber in Kosmetika Verwendung. Styrole eignen sich zur Herstellung von Polymeren. Aromatische Olefine eignen sich insbesondere noch zur Herstellung von Pharmaka, Herbiziden, Fungiziden und Riechstoffen.

Die bisher bekannten Verfahren zur Olefinierung von Aryldiazoniumsalzen verwenden generell homogene Palladiumkatalysatoren. Die vergleichbare Olefinierung von Arylbromiden und Arylchloriden gelingt ebenfalls mittels homogenen Palladiumverbindungen. In einigen Fällen erzielt man auch eine Reaktion mit ausgewählten heterogenen Palladiumkatalysatoren. Die Ausbeuten sind dann jedoch üblicherweise niedriger als mit homogenen Katalysatoren; in vielen Fällen gelingt die Umsetzung überhaupt nicht. Insofern war es überraschend, daß man im erfindungsgemäßen Verfahren heterogene Palladiumkatalysatoren ebenso effektiv wie homogene Palladiumverbindungen einsetzen kann.

Das erfindungsgemäße vereinfachte Verfahren weist folgende Vorteile auf:

Es stellt eine einfache, allgemeine Methode dar, die es erlaubt, Aryldiazoniumsalze mit Olefinen so umzusetzen, daß aromatische Olefine technisch besser als bisher erhältlich sind. Ein bedeutender Vorteil liegt darin, daß das Katalysatorsystem heterogen vorliegt und somit durch einfaches Abfiltrieren ein Recycling des wertvollen Palladiumkatalysators möglich ist. Ein weiterer Vorteil besteht darin, daß auf den Zusatz von Basen verzichtet werden kann und damit kein Salzabfall anfällt, was ökologisch günstig ist.

Die nachstehenden Beispiele dienen zur Erläuterung des erfindungsgemäßen Verfahrens, ohne es darauf zu beschränken.

### Beispiel 1

7,0 g (31,5 mmol) p-Methoxyphenyldiazoniumtetrafluoroborat und 6,3 g (63,0 mmol) Acrylsäureethylester wurden in 40 ml Ethanol (98 %ig) suspendiert. Die erhaltene Suspension wurde mit 0,325 g (0,16 mmol) Palladium auf Aktivkohle (5 %ig) bei 0 °C versetzt. Darauf wurde die Reaktionsmischung binnen einer Stunde auf 60 °C erwärmt und 12 Stunden bei dieser Temperatur gerührt. Nach Abkühlung auf Raumtemperatur wurde der Katalysator abfiltriert und mit Ethanol gewaschen. Anschliessend wurde mit 100 ml Dichlormethan verdünnt und dreimal mit 60 ml Wasser gewaschen. Die organische Phase wurde im Vakuum eingeengt. Man erhielt 8,0 g Rohprodukt, das nach GC- und NMR-spektroskopischen Analysen 85 % p-Methoxyzimtsäureethylester enthielt.
Ausbeute: 98 % (d.Th.) p-Methoxyzimtsäureethylester

### Beispiel 2

Es wurde, wie in Beispiel 1 beschrieben, verfahren, wobei jedoch als Katalysatorsystem 1 mol% Palladium auf Aluminiumoxid (5 %ig) eingesetzt wurde.
Ausbeute: 89 % (d. Th.) p-Methoxyzimtsäureethylester d.Th.

### Beispiel 3

Es wurde, wie in Beispiel 1 beschrieben, verfahren, wobei jedoch als Katalysatorsystem 1 mol% Palladium auf Bariumsulfat (5 %ig) eingesetzt wurde.
Ausbeute: 94 % (d.Th.) p-Methoxyzimtsäureethylester.

### Beispiel 4

Es wurde, wie in Beispiel 1 beschrieben, verfahren, wobei jedoch als Katalysatorsystem 1 mol% Palladium auf Aktivkohle (5 %ig) eingesetzt wurde. Als Ausgangsverbindungen wurden 7,0 g (31,5 mmol) p-Methoxyphenyldiazoniumtetrafluoroborat und 4,5 g (63,0 mmol) Acrylsäure, suspendiert in 40 ml Methanol, verwendet.
Ausbeute: 72 % (d.Th.) p-Methoxyzimtsäuremethylester.

### Beispiel 5

Es wurde, wie in Beispiel 1 beschrieben, verfahren, wobei jedoch als Katalysator 1 mol% Palladium auf Aktivkohle (5%ig) eingesetzt wurde.
Ausgangsverbindungen waren 7,0 g (31,5 mmol) p-Methoxyphenyldiazoniumtetrafluoroborat und 5,4 g (63,0 mmol) Acrylsäuremethylester, suspendiert in 40 ml Methanol.
Ausbeute: 95 % (d.Th.) p-Methoxyzimtsäuremethylester

### Beispiel 6

Es wurde, wie in Beispiel 1 beschrieben, verfahren, wobei jedoch als Katalysator 1 mol% Palladium auf Aktivkohle (5 %ig) eingesetzt wurde.
Ausgangsverbindungen waren 6,5 g (31,5 mmol) 3-Methylphenyldiazoniumtetrafluoroborat und 6,3 g (63,0 mmol) Acrylsäureethylester, suspendiert in 40 ml Ethanol.
Ausbeute: 89 % (d.Th.) 3-Methylzimtsäureethylester

### Beispiel 7

Es wurde, wie in Beispiel 1 beschrieben, verfahren, wobei jedoch als Katalysator 1 mol% Palladium auf Aktivkohle (5 %ig) eingesetzt wurde.
Ausgangsverbindungen waren 7,1 g (31,5 mmol) 4-Chlorphenyldiazoniumtetrafluoroborat und 6,3 g (63 mmol) Acrylsäureethylester, suspendiert in 40 ml Ethanol .
Ausbeute: 92 % (d.Th.) 4-Chlorzimtsäureethylester.

### Beispiel 8

7,0 g (31,5 mmol) p-Methoxyphenyldiazoniumtetrafluoroborat, 11,61 g (63,0 mmol) Acrylsäure-2-ethylhexylester werden in 40 ml Dimethylsulfoxid suspendiert und mit 0,75 g (0,32 mmol) Palladium auf Aktivkohle (5 %ig) bei 0°C versetzt. Die Reaktionsmischung wurde binnen einer Stunde auf 60°C erwärmt und 12 Stunden bei dieser Temperatur gerührt.
Nach Abkühlung auf Raumtemperatur wurde der Katalysator abfiltriert und mit Ethanol gewaschen. Anschließend wurde mit 100 ml Dichlormethan verdünnt und dreimal mit 60 ml Wasser gewaschen. Die organische Phase wurde im Vakuum eingeengt. Das erhaltene Rohprodukt wurde säulenchromatographiert.
Ausbeute: 80 % p-Methoxyzimtsäure-2-ethylhexylester.

### Vergleichsversuch

Man vermischt unter Rühren 12,3 g 4-Anisidin (4-Methoxyanilin) mit 10 ml konzentrierter Schwefelsäure und 60 ml 2-Ethylhexanol und kühlt die Mischung auf etwa 10°C ab. Anschließend gibt man langsam 11,7 g Amylnitrit zu und rührt nach beendeter Zugabe noch 40 Minuten. Das Reaktionsgemisch wird nachfolgend mit 20,2 g Acrylsäure-2-ethylhexylester und 100 mg Pd (5 % Pd auf Aktivkohle) versetzt und binnen 1 Stunde auf 65°C erwärmt und 12 Stunden bei dieser Temperatur gerührt. Man verdünnt mit 100 ml Wasser und extrahiert mit 200 ml Dichlormethan. Wie Untersuchungen mittels Dünnschichtchromatographie und Gaschromatographie zeigen, hat sich p-Methoxyzimtsäure-2-ethylhexylester nicht einmal in kleinsten Mengen gebildet. Der eingesetzte Acrylsäure-2-ethylhexylester liegt im wesentlichen in unveränderter Form vor.

## Patentansprüche

1. Verfahren zur Herstellung aromatischer Olefine der allgemeinen Formel (I) 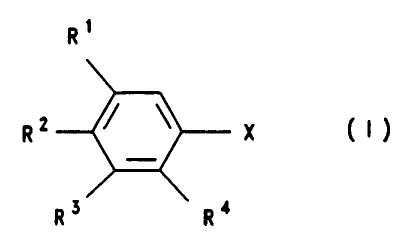 in welcher R¹, R², R³, unabhängig voneinander, Wasserstoff, Alkyl(C₁-C₈), Alkoxy (C₁-C₅), Phenyl, Fluor, Chlor, Brom, -OH, -NO₂, -CN, -CHO, - CO(Alkyl)(C₁-C₄), -CO Phenyl, -COO Alkyl(C₁-C₄), -OCO Alkyl(C₁-C₄), -NHCO Alkyl(C₁-C₄), -CF₃, -NH₂, -NH Alkyl(C₁-C₄) oder -N(Alkyl(C₁-C₄))₂ und
R⁴ Wasserstoff, Alkyl(C₁-C₈), Phenyl, Fluor, Chlor, Brom, -OH,- NO₂, -CN, -CHO oder -OCO Alkyl(C₁-C₄) bedeuten, wobei die Alkyl- und Alkoxygruppen geradkettig oder verzweigt sein können,
X Alkenyl (C₂-C₁₂), Cycloalkenyl(C₄-C₈) oder die Gruppierung 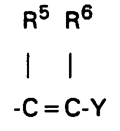 bedeutet, worin R⁵ und R⁶ unabhängig voneinander Wasserstoff oder Methyl, und Y -Phenyl, -CN, -COOH, -COO Alkyl(C₁-C₁₂), -COO Phenyl,
- CON(Alkyl(C₁-C₁₂))₂, -CONHAlkyl(C₁-C₁₂), 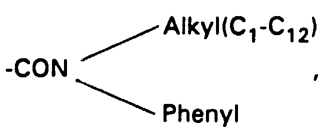 - CONH₂, -CON(Phenyl)₂, -CO Alkyl(C₁-C₁₂), -CO Phenyl, -O Alkyl(C₁-C₁₂) oder -O Phenyl bedeutet oder den Rest 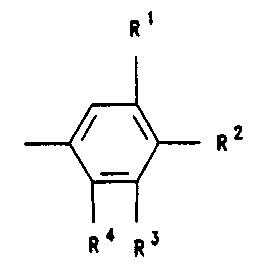 darstellt, worin R¹ - R⁴ die vorstehend genannten Bedeutungen haben, dadurch gekennzeichnet, daß man ein Aryldiazoniumsalz der allgemeinen Formel (2) 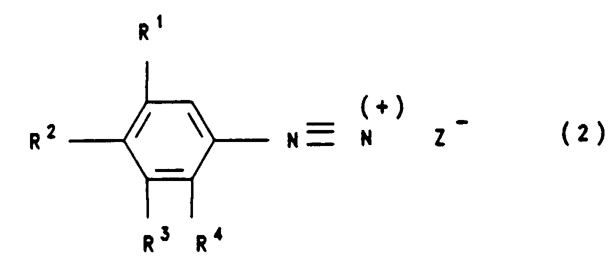 in welcher R¹, R², R³, R⁴ die vorstehend genannten Bedeutungen haben und Z das Äquivalent eines Anions einer organischen oder anorganischen Säure eines pkₐ-Wertes kleiner als 7 darstellt, mit einem Olefin der allgemeinen Formel (3)
HX (3)
in welcher X die vorstehend genannte Bedeutung hat, in Gegenwart katalytischer Mengen eines heterogenen Palladiumkatalysators in einem organischen Lösungsmittel bei Temperaturen von -20 °C bis 150 °C umsetzt.

2. Verfahren nach Anspruch 1, dadurch gekennzeichnet, daß man bei Temperaturen von 0 °C bis 100 °C umsetzt.

3. Verfahren nach mindestens einem der Ansprüche 1 und 2, dadurch gekennzeichnet, daß das Trägermaterial des heterogenen Palladiumkatalysators aus Aktivkohle, Aluminiumoxid, Bariumsulfat, Bimsstein, Tonerde, Kieselgur oder Kieselgel besteht.

4. Verfahren nach mindestens einem der Ansprüche 1 bis 3, dadurch gekennzeichnet, daß das Trägermaterial des heterogenen Palladiumkatalysators aus Aktivkohle, Aluminiumoxid oder Bariumsulfat besteht.

5. Verfahren nach mindestens einem der Ansprüche 1 bis 4, dadurch gekennzeichnet, daß der eingesetzte heterogene Palladiumkatalysator 1 bis 20 Gew.-% Palladium, bezogen auf das Trägermaterial enthält.

6. Verfahren nach mindestens einem der Ansprüche 1 bis 5, dadurch gekennzeichnet, daß der eingesetzte heterogene Palladiumkatalysator 2 bis 10 Gew.-% Palladium, bezogen auf das Trägermaterial, enthält.

7. Verfahren nach mindestens einem der Ansprüche 1 bis 6, dadurch gekennzeichnet, daß man 0,001 bis 20 mol% Palladium, bezogen auf das Aryldiazoniumsalz, einsetzt.

8. Verfahren nach mindestens einem der Ansprüche 1 bis 7, dadurch gekennzeichnet, daß man 0,1 bis 1 mol% Palladium, bezogen auf das Aryldiazoniumsalz, einsetzt.

9. Verfahren nach mindestens einem der Ansprüche 1 bis 8, dadurch gekennzeichnet, daß man 1 mol Aryldiazoniumsalz mit 0,2 bis etwa 10 mol Olefin umsetzt.

10. Verfahren nach mindestens einem der Ansprüche 1 bis 9, dadurch gekennzeichnet, daß man 1 mol Aryldiazoniumsalz mit 0,4 bis etwa 5 mol Olefin umsetzt.

11. Verfahren nach einem der Ansprüche 1 bis 10, dadurch gekennzeichnet, daß man etwa 1 mol Aryldiazoniumsalz mit 0,6 bis 4 mol Olefin umsetzt.

12. Verfahren nach mindestens einem der Ansprüche 1 bis 11, dadurch gekennzeichnet, daß man Verbindungen der in Anspruch 1 genannten allgemeinen Formel (3) umsetzt, in deren Gruppierung 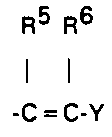 mindestens einen der Reste R⁵ und R⁶ ein Wasserstoffatom darstellt und Y eine COOH, COOMethyl, COOEthyl, COOPropyl, COOButyl oder COO-2-Ethyl-hexylgruppe bedeutet.

13. Verfahren nach mindestens einem der Ansprüche 1 bis 11, dadurch gekennzeichnet, daß man Verbindungen der in Anspruch 1 genannten allgemeinen Formel (3) umsetzt, in welcher X eine Alkenyl(C₂-C₈)-, Cyclopentenyl- oder Cyclohexenylgruppe bedeutet.

14. Verfahren nach mindestens einem der Ansprüche 1 bis 11, dadurch gekennzeichnet, daß man als Verbindung der in Anspruch 1 genannten allgemeinen Formel (3) Styrol, Ethylen, Propylen, Acrylsäuremethyl-, -ethyl-, -propyl-, -butyl- oder -2-ethylhexylester mit 4-Methoxy-, 4-Acetyl-, 4-Formyl-, 4-Nitro- oder 4-Cyanobenzoldiazoniumsalz umsetzt.

15. Verfahren nach mindestens einem der Ansprüche 1 bis 11, dadurch gekennzeichnet, daß man als Verbindung der in Anspruch 1 genannten allgemeinen Formel (3) Styrol oder Ethylen mit 4-Cyanobenzoldiazoniumsalz umsetzt.

16. Verfahren nach mindestens einem der Ansprüche 1 bis 15, dadurch gekennzeichnet, daß man Verbindungen der in Anspruch 1 genannten allgemeinen Formel (2) umsetzt, in welcher Z BF₄⁻, HSO₄⁻, PF₆⁻, Cl⁻, SO₄²⁻, CH₃COO⁻, Aryl-SO₃⁻, wobei der Arylrest substituiert sein kann, darstellt.

## Claims

1. A process for preparing aromatic olefins of the formula I 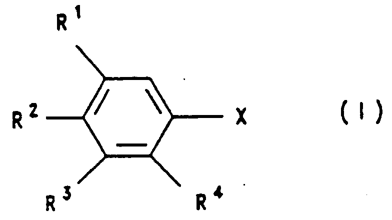 in which R¹, R² and R³ are, independently of one another, hydrogen, alkyl (C₁-C₈) , alkoxy(C₁-C₅), phenyl, fluorine, chlorine, bromine, -OH, -NO₂, -CN, -CHO, -COalkyl(C₁-C₄), -COphenyl, -COOalkyl(C₁-C₄), -OCOalkyl(C₁-C₄), -NHCOalkyl(C₁-C₄), -CF₃, -NH₂, -NHalkyl(C₁-C₄) or -N(alkyl(C₁-C₄))₂ and
R⁴ is hydrogen, alkyl(C₁-C₈), phenyl, fluorine, chlorine, bromine, -OH, -NO₂, -CN, -CHO or -OCOalkyl(C₁-C₄), where the alkyl and alkoxy groups may be straight-chain or branched, and
X is alkenyl(C₂-C₁₂), cycloalkenyl(C₄-C₈) or the group 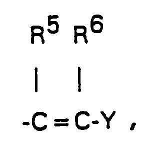 in which R⁵ and R⁶ are, independently of one another, hydrogen or methyl, and Y is -phenyl, -CN, -COOH, -COOalkyl (C₁-C₁₂), -COOphenyl, -CON(Alkyl(C₁-C₁₂))₂, -CONHAlkyl(C₁-C₁₂), 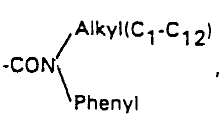 -CONH₂, -CON(phenyl)₂, -COalkyl(C₁-C₁₂), -COphenyl, -Oalkyl(C₁-C₁₂), -Ophenyl or the radical 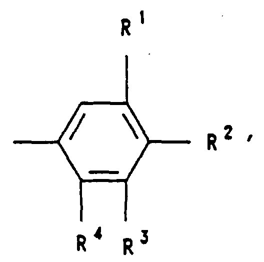 in which R¹-R⁴ are as defined above, which comprises reacting an aryldiazonium salt of the formula (2) 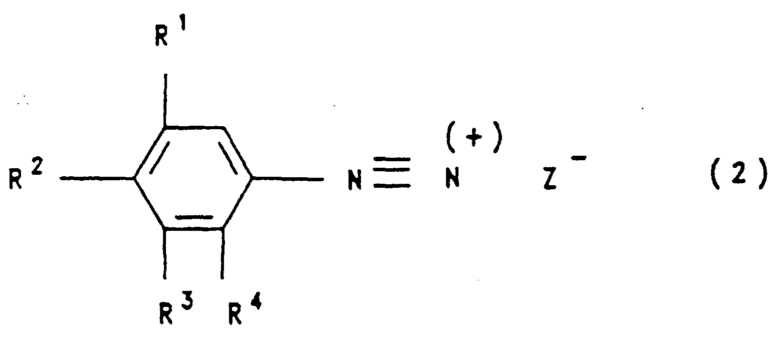 in which R¹, R², R³ and R⁴ are as defined above and Z is the equivalent of an anion of an organic or inorganic acid having a pKₐ of less than 7, with an olefin of the formula (3)
HX (3)
in which X is as defined above, in the presence of catalytic amounts of a heterogeneous palladium catalyst in an organic solvent at temperatures from -20°C to 150°C.

2. The process as claimed in claim 1, wherein the reaction is carried out at temperatures from 0°C to 100°C.

3. The process as claimed in at least one of claims 1 and 2, wherein the support material of the heterogeneous palladium catalyst comprises activated carbon, aluminum oxide, barium sulfate, pumice, alumina, kieselguhr or silica gel.

4. The process as claimed in at least one of claims 1 to 3, wherein the support material of the heterogeneous palladium catalyst comprises activated carbon, aluminum oxide or barium sulfate.

5. The process as claimed in at least one of claims 1 to 4, wherein the heterogeneous palladium catalyst used contains from 1 to 20% by weight of palladium, based on the support material.

6. The process as claimed in at least one of claims 1 to 5, wherein the heterogeneous palladium catalyst used contains from 2 to 10% by weight of palladium, based on the support material.

7. The process as claimed in at least one of claims 1 to 6, wherein from 0.001 to 20 mol% of palladium, based on the aryldiazonium salt, is used.

8. The process as claimed in at least one of claims 1 to 7, wherein from 0.1 to 1 mol% of palladium, based on the aryldiazonium salt, is used.

9. The process as claimed in at least one of claims 1 to 8, wherein 1 mol of aryldiazonium salt is reacted with from 0.2 to 10 mol of olefin.

10. The process as claimed in at least one of claims 1 to 9, wherein 1 mol of aryldiazonium salt is reacted with from 0.4 to 5 mol of olefin.

11. The process as claimed in at least one of claims 1 to 10, wherein 1 mol of aryldiazonium salt is reacted with from 0.6 to 4 mol of olefin.

12. The process as claimed in at least one of claims 1 to 11, wherein the reaction is carried out of compounds of the formula (3) specified in claim 1 in whose grouping 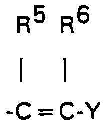 at least one of the radicals R⁵ and R⁶ is a hydrogen atom and Y is a COOH, COOmethyl, COOethyl, COOpropyl, COObutyl or COO-2-ethylhexyl group.

13. The process as claimed in at least one of claims 1 to 11, wherein the reaction is carried out of compounds of the formula (3) specified in claim 1 in which X is an alkenyl(C₂-C₈), cyclopentenyl or cyclohexenyl group.

14. The process as claimed in at least one of claims 1 to 11, wherein the compound of the formula (3) specified in claim 1 is styrene, ethylene, propylene, methyl acrylate, ethyl acrylate, propyl acrylate, butyl acrylate or 2-ethylhexyl acrylate and is reacted with 4-methoxy-, 4-acetyl-, 4-formyl-, 4-nitro- or 4-cyanobenzenediazonium salt.

15. The process as claimed in at least one of claims 1 to 11, wherein the compound of the formula (3) specified in claim 1 is styrene or ethylene and is reacted with 4-cyanobenzenediazonium salt.

16. The process as claimed in at least one of claims 1 to 15, wherein the reaction is carried out of compounds of the formula (2) specified in claim 1 in which Z is BF₄⁻, HSO₄⁻, PF₆⁻, Cl⁻, SO₄²⁻, CH₃COO⁻ or aryl-SO₃⁻ in which the aryl radical may be substituted.

## Revendications

1. Procédé pour la préparation d'oléfines aromatiques de formule générale I 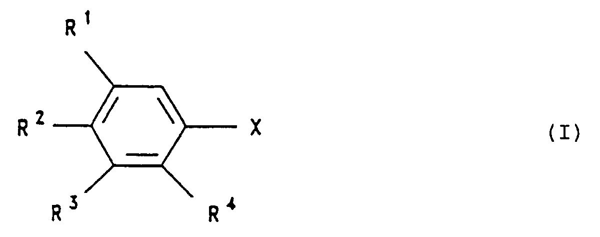 dans laquelle, R¹, R², R³, indépendamment les uns des autres, représentent l'hydrogène, alkyle en C₁-C₈, alcoxy en C₁-C₅, phényle, le fluor, le chlore, le brome, -OH, -NO₂, -CN, -CHO, -CO(alkyle en C₁-C₄),
-CO phényle, -COO(alkyle en C₁-C₄), -OCO(alkyle en C₁-C₄), -NHCO(alkyle en C₁-C₄), -CF₃, -NH₂, -NH(alkyle en C₁-C₄) ou -N(alkyle en C₁-C₄)₂ et
R⁴ représente l'hydrogène, alkyle en C₁-C₈, phényle, le fluor, le chlor, le brome, -OH, -NO₂, -CN, -CHO ou -OCO (alkyle en C₁-C₄), le groupe alkyle et le groupe alcoxy pouvant être linéaire ou ramifié,
X représente alcényle en C₂-C₁₂, cycloalcényle en C₄-C₈ ou le groupement 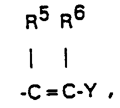 où R⁵ et R⁶, indépendamment l'un de l'autre, représentent l'hydrogène ou méthyle, et Y représente phényle, -CN, -COOH, -COO(alkyle en C₁-C₁₂), -COO-phényle, -CON(alkyle en C₁-C₁₂)₂, -CONH(alkyle en C₁-C₁₂), 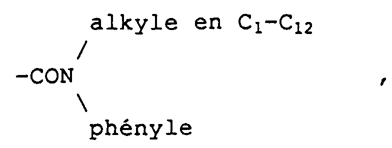 CONH₂, CON(phényle)₂, -CO(alkyle en C₁-C₁₂), -CO-phényle, -O-alkyle en C₁-C₁₂, -O-phényle ou le radical de formule 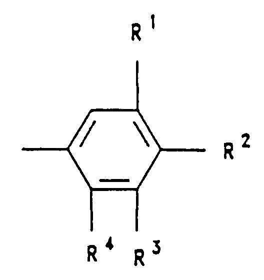 dans laquelle R¹ à R⁴ ont les significations données précédemment, en faisant réagir un sel d'aryldiazonium de formule générale (2) 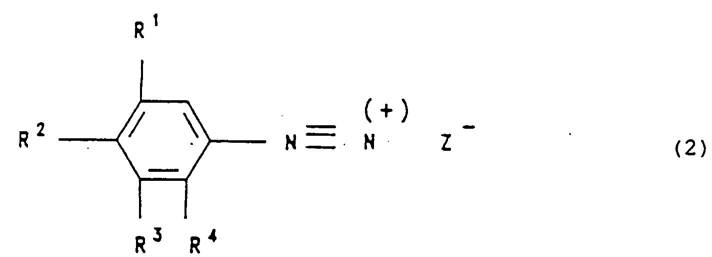 dans laquelle R¹, R², R³, R⁴ ont les significations données précédemment et Z est l'équivalent d'un anion d'un acide organique ou inorganique avec une valeur pKₐ inférieure à 7, avec une oléfine de formule générale (3)
HX (3)
dans laquelle X a la signification donnée précédemment, en présence de quantités catalytiques d'un catalyseur hétérogène au palladium dans un solvant organique à une température de -20 °C à 150 °C, de préférence de 0 °C à 100 °C.

2. Procédé selon la revendication 1, caractérisé en ce que l'on effectue la réaction à une température de 0 à 100 °C.

3. Procédé selon au moins l'une des revendications 1 et 2, caractérisé en ce que la matière de support du catalyseur hétérogène au palladium est le charbon actif, l'oxyde d'aluminium, le sulfate de baryum, la pierre ponce, l'alumine, le kieselgur ou le gel de silice.

4. Procédé selon au moins l'une des revendications 1 à 3, caractérisé en ce que la matière de support du catalyseur hétérogène au palladium est le charbon actif, l'oxyde d'aluminium ou le sulfate de baryum.

5. Procédé selon au moins l'une des revendications 1 à 4, caractérisé en ce que le catalyseur hétérogène au palladium utilisé contient de 1 à 20 % en poids de palladium par rapport à la matière de support.

6. Procédé selon au moins l'une des revendications 1 à 5, caractérisé en ce que le catalyseur hétérogène au palladium utilisé contient de 2 à 10 % en poids de palladium par rapport à la matière de support.

7. Procédé selon au moins l'une des revendications 1 à 6, caractérisé en ce que l'on utilise de 0,001 à 20 % en moles de palladium par rapport au sel d'aryldiazonium.

8. Procédé selon au moins l'une des revendications 1 à 7, caractérisé en ce que l'on utilise de 0,1 à 1 % en moles de palladium par rapport au sel d'aryldiazonium.

9. Procédé selon au moins l'une des revendications 1 à 8, caractérisé en ce que l'on fait réagir 1 mole de sel d'aryldiazonium avec 0,2 à environ 10 moles d'oléfine.

10. Procédé selon au moins l'une des revendications 1 à 9, caractérisé en ce que l'on fait réagir 1 mole de sel d'aryldiazonium avec 0,4 à environ 5 moles d'oléfine.

11. Procédé selon au moins l'une des revendications 1 à 10, caractérisé en ce que l'on fait réagir 1 mole de sel d'aryldiazonium avec 0,6 à environ 4 moles d'oléfine.

12. Procédé selon au moins l'une des revendications 1 à 11, caractérisé en ce que l'on fait réagir des composés de formule générale (3) citée dans la revendication 1, dans laquelle dans le groupement 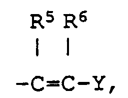 au moins l'un des radicaux R⁵ et R⁶, représente un atome d'hydrogène, et Y représente -COOH, COO-méthyle, COO-éthyle, COO-propyle, -COO-butyle ou COO-2-éthylhexyle.

13. Procédé selon au moins l'une des revendications 1 à 11, caractérisé en ce que l'on fait réagir des composés de formule générale (3) citée dans la revendication 1 dans laquelle X représente un groupe alcényle en C₂-C₈, cyclopentényle ou cyclohexényle.

14. Procédé selon au moins l'une des revendications 1 à 11, caractérisé en ce que l'on fait réagir comme composés de formule générale (3) citée dans la revendication 1 le styrène, l'éthylène, le propylène, l'acrylate de méthyle, d'éthyle, de propyle, de butyle ou de 2-éthylhexyle avec le sel de 4-méthoxy-, de 4-acétyl-, de 4-formyl-, de 4-nitro ou de 4-cyano-benzènediazonium.

15. Procédé selon au moins l'une des revendications 1 à 11, caractérisé en ce que l'on fait réagir comme composés de formule générale (3) citée dans la revendication 1 le styrène ou l'éthylène avec le sel de 4-cyanobenzènediazonium.

16. Procédé selon au moins l'une des revendications 1 à 15, caractérisé en ce que l'on fait réagir des composés de formule générale (2) citée dans la revendication 1 dans lesquels Z représente BF₄⁻, HSO₄⁻, PF₆⁻, Cl⁻, SO₄²⁻, CH₃COO⁻, aryl-SO₃⁻, le radical aryle pouvant être substitué.
